(19) Europäisches Patentamt
European Patent Office
Office européen des brevets

(11) EP 0 837 680 B1

(12) **EUROPEAN PATENT SPECIFICATION**

(45) Date of publication and mention of the grant of the patent:
**31.01.2001 Bulletin 2001/05**

(21) Application number: **96921928.6**

(22) Date of filing: **04.06.1996**

(51) Int Cl.7: **A61K 31/44**

(86) International application number:
**PCT/EP96/02430**

(87) International publication number:
**WO 96/41629 (27.12.1996 Gazette 1996/56)**

(54) **USE OF PHENOXY PYRIDINE DERIVATIVES FOR THE TREATMENT OF ILLNESSES CAUSED BY DISORDERS OF THE DOPAMINE SYSTEM**

VERWENDUNG VON PHENOXYPYRIDINDERIVATEN ZUR BEHANDLUNG DER DURCH STOERUNGEN DES DOPAMINSYSTEMS VERURSACHTEN ERKRANKUNGEN

UTILISATION DE DERIVES DE PHENOXY PYRIDINE DANS LE TRAITEMENT DE PATHOLOGIES INDUITES PAR DES TROUBLES DU SYSTEME DOPAMINERGIQUE

(84) Designated Contracting States:
**AT BE CH DE DK ES FI FR GB GR IE IT LI LU NL PT SE**

(30) Priority: **09.06.1995 CH 170495**

(43) Date of publication of application:
**29.04.1998 Bulletin 1998/18**

(73) Proprietor: **F. HOFFMANN-LA ROCHE AG**
**4070 Basel (CH)**

(72) Inventors:
- **GODEL, Thierry**
**CH-4056 Basle (CH)**
- **HARTMAN, Deborah**
**CH-4058 Basle (CH)**
- **RIEMER, Claus**
**D-79418 Schliengen (DE)**

(74) Representative: **Poppe, Regina**
**P.O. Box 3255**
**4002 Basle (CH)**

(56) References cited:
**CH-A- 474 511** **DE-A- 1 964 421**
**FR-M- 6 263**

- **DATABASE WPI Section Ch, Week 8242 Derwent Publications Ltd., London, GB; Class B02, AN 82-88836E XP002017360 & JP,A,57 145 872 (OTSUKA PHARM KK) , 9 September 1982 & CHEMICAL ABSTRACTS, vol. 98, no. 19, 9 May 1983 Columbus, Ohio, US; abstract no. 160600,**
- **JOURNAL OF MEDICINAL CHEMISTRY, vol. 15, no. 3, 1972, pages 286-291, XP002017359 S.N. RASTOGI ET AL.: "AGENTS ACTING ON THE CENTRAL NERVOUS SYSTEM"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 837 680 B1

## Description

The invention is concerned with aromatic ethers, especially phenoxy-pyridine derivatives of the general formula

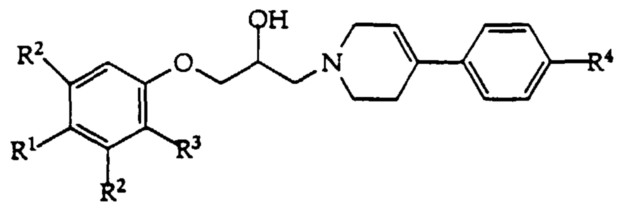

I

wherein

- $R^1$ signifies hydrogen, lower-alkyl, halogen, lower-alkoxy or nitro;
- $R^2$ signifies hydrogen, lower alkyl or trifluoromethyl; and
- $R^3$ signifies hydrogen, lower alkyl, trifluoromethyl, benzyl, hydroxy-lower-alkyl, lower alkoxy, lower-alkyl-carbonyl-amino, carbonyl-lower-alkyl, benzyl, di-lower-alkyl-amino-carbonyl, carbonyl-amino or amino-carbonyl-amino; or
- $R^2$ and $R^3$ together optionally signify a fused benzene ring and
- $R^4$ signifies hydrogen or halogen,

as well as pharmaceutically acceptable salts of compounds of general formula I.

These compounds and salts are known. The preparation of the aforementioned compounds and their use as antiinflammatory, antiallergic, antitussive and analgesic agents is described in DE-OS 1964421.

As used herein, the term "lower alkyl" denotes straight or branched chain lower alkyl of one to six carbon atoms, for example, methyl, ethyl, isopropyl, butyl, pentyl and the like. The term "lower alkoxy" denotes lower alkyl ether groups in which the lower alkyl is as described above, for example, methoxy, ethoxy, isopropoxy and the like. The term "halogen" denotes chlorine, bromine, fluorine and iodine. Of the halogen atoms, fluorine and chlorine are preferred.

It has now surprisingly been found that these compounds can be used in the control or prevention of illnesses which are caused by disorders of the dopamine system. Thereto there belong psychotic illnesses such as e.g. schizophrenia. The object of the present invention is accordingly the use of compounds of formula I for the manufacture of a medicament for the treatment or prevention of psychotic illnesses which are caused by disorders of the dopamine system and the use of these compounds as active ingredients in the production of medicaments for the said purpose.

The novel pharmacological properties of these compounds follow from a high selective affinity to a neuroreceptor, especially to the dopamine-D4 receptor. Thereby, it can be expected that when these compounds are used significantly fewer side effects will occur than in the case of known classical neuroleptic agents, e.g. haloperidol, which, as is known, bind to the $D_2$ or $D_3$ receptor. It has been found that in the case of schizophrenia the $D_2$ and $D_3$ receptor density increases by about 10%, while it can increase in the case of the $D_4$ receptor by about 600% (TiPS, July 1994, vol. 15, p. 264-70).

<u>Test description</u>

The compounds were characterized by their binding behaviour at the $D_4$ receptor.

Compounds, which, moreover, were expected to have a good selectivity, were compared with respect to their $D_2$ receptor activity.
CHO cells (Chinese Hamster Ovary) were used in the test.
Crude membranes were isolated by ultracentrifugation from $D_4$-CHO and $D_2$-CHO cells and were stored at -80°C. After thawing and homogenizing in a buffer solution (50 mM Tris, 1 mM EDTA, 5 mM KCl, 1.5 mM $CaCl_2$, 4 mM $MgCl_2$, pH 7.4) they were incubated at room temperature for 90 minutes with 200 pM [$^3$H]-spiperone and an increasing concentration ($1 \times 10^{-11}$ M to $1 \times 10^{-4}$ M) of test compound. A non-specific binding was established by incubating in the presence of $1 \times 10^{-5}$ M (+)-butaclamol. The unbound radioligand was removed by filtration through a GF/C glass filter and the bound radioactivity was determined by scintillation in a Packard TopCount.

The following Table shows the binding behaviour of some selected compounds at the $D_4$ receptor.
The Ki value is a binding constant which shows the affinity of the compounds to the $D_4$ receptor. It was determined

using $^3H$-spiperone. The calculation of the value was effected with ligand.

Table 1

| Cpd. No. | $R^1$ | $R^2$ | $R^3$ | $R^4$ | Ki at $D_4$ [nM] |
|---|---|---|---|---|---|
| 1 | H | $CH_3$ | H | H | < 100 |
| 2 | H | H | $CH_3$ | H | < 100 |
| 3 | H | H | $CF_3$ | H | < 100 |
| 4 | H | H | $-CH_2OH$ | H | < 100 |
| 5 | H | H | $-OCH_3$ | H | < 100 |
| 6 | H | H | $-NHCOCH_3$ | H | < 100 |
| 7 | H | H | $-COCH_3$ | H | < 100 |
| 8 | $CH_3$ | $CH_3$ | H | H | < 100 |
| 9 | Cl | H | $-NHCOCH_3$ | H | < 100 |
| 10 | H | $R^2$ and $R^3$ together benzene ring | | H | < 100 |
| 11 | H | $CF_3$ | H | F | < 100 |
| 12 | H | H | $-CONH_2$ | F | < 100 |
| 13 | H | H | $-NHCONH_2$ | F | < 100 |
| 14 | H | H | $-NHCOCH_3$ | Cl | < 100 |

Particularly active compounds are set forth in Table 1.
These are the following compounds:
1 1-(4-Phenyl-3,6-dihydro-2H-pyridin-1-yl)-3-m-tolyloxypropan-2-ol
2 1-(4-Phenyl-3,6-dihydro-2H-pyridin-1-yl)-3-o-tolyloxypropan-2-ol
3 1-(4-Phenyl-3,6-dihydro-2H-pyridin-1-yl)-3-(3-trifluoromethylphenoxy)-propan-2-ol
4 1-(2-Hydroxymethyl-phenoxy)-3-(4-phenyl-3,6-dihydro-2H-pyridin-1-yl)-propan-2-ol
5 1-(2-Methoxy-phenoxy)-3-(4-phenyl-3,6-dihydro-2H-pyridin-1-yl)-propan-2-ol
6 N-[2-[2-Hydroxy-3-(4-phenyl-3,6-dihydro-2H-pyridin-1-yl)-propoxy]-phenyl]-acetamide
7 1-[2-[2-Hydroxy-3-(4-phenyl-3,6-dihydro-2H-pyridin-1-yl)-propoxy]-phenyl]-ethanone
8 1-(3,4-Dimethyl-phenoxy)-3-(4-phenyl-3,6-dihydro-2H-pyridin-1-yl)-propan-2-ol
9 N-[5-Chloro-2-[2-hydroxy-3-(4-phenyl-3,6-dihydro-2H-pyridin-1-yl)-propoxy]-phenyl]-acetamide
10 1-(Naphthalen-1-yloxy)-3-(4-phenyl-3,6-dihydro-2H-pyridin-1-yl)-propan-2-ol
11 1-[4-(4-Fluoro-phenyl)-3,6-dihydro-2H-pyridin-1-yl)-3-(3-trifluoromethyl-phenoxy)-propan-2-ol,
12 2-[3-[4-(4-Fluoro-phenyl)-3,6-dihydro-2H-pyridin-1-yl]-2-hydroxy-propoxy]-benzamide,
13 [2-[3-[4-(4-Fluoro-phenyl)-3,6-dihydro-2H-pyridin-1-yl]-2-hydroxy-propoxy]-phenyl]-urea
14 N-[2-[3-]4-(4-Chloro-phenyl)-3,6-dihydro-2H-pyridin-1-yl)-2-hydroxy-propoxy]-phenyl]-acetamide.

The compounds of formula I and pharmaceutically acceptable salts thereof can be used as medicaments, e. g. in the form of pharmaceutical preparations. The pharmaceutical preparations can be administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions.

The compounds of formula I and pharmaceutically acceptable salts thereof can be processed with pharmaceutically inert, inorganic or organic carriers for the production of pharmaceutical preparations. Lactose, corn starch or derivatives thereof, talc, stearic acid or its salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like; depending on the nature of the active ingredient no carriers are, however, usually required in the case of soft gelatine capsules. Suitable carriers for the production of solutions and syrups are, for example, water, polyols, sucrose, invert sugar, glucose and the like. Adjuvants such as alcohols, polyols, glycerol, vegetable oils and the like can be used for aqueous injection solutions of water-soluble salts of compounds of formula I, but as a rule are not necessary. Suitable carriers for suppositories are, for example, natural or hardened oils, waxes, fats, semi-liquid or liquid polyols and the like.

The pharmaceutical preparations can, moreover, contain preservatives, solubilizers, stabilizers, wetting agents, emulsifiers, sweeteners, colorants, flavorants, salts for varying the osmotic pressure, buffers, coating agents or antioxidants. They can also contain still other therapeutically valuable substances.

As mentioned earlier, the use of a compound of formula I in the manufacture of medicaments containing a

compound of formula I or a pharmaceutically acceptable salt thereof and a therapeutically inert excipient for treating psychotic illnesses caused by disorders of the dopamine system are also an object of the present invention, as is a process for the production of such medicaments for the said purpose which comprises bringing one or more compounds of formula I or pharmaceutically acceptable salts thereof and, if desired, one or more other therapeutically valuable substances into a galenical administration form together with one or more therapeutically inert carriers. The dosage can vary within wide limits and will, of course, be fitted to the individual requirements in each particular case. In general, a daily dosage of 1 mg to 1000 mg should be appropriate.

Finally, as mentioned earlier, the use of compounds of formula I and of pharmaceutically usable salts thereof for the production of medicaments, especially for the control or prevention of psychotic illnesses which are caused by disorders of the dopamine system, is the object of the invention.

Example A

Tablets of the following composition are produced in the usual manner:

| | | mg/tablet |
|---|---|---|
| Active ingredient | | 100 |
| Powd. lactose | | 95 |
| White corn starch | | 35 |
| polyvinylpyrrolidone | | 8 |
| Na carboxymethylstarch | | 10 |
| Magnesium stearate | | 2 |
| | Tablet weight | 250 |

Example B

Tablets of the following composition are produced in the usual manner:

| | | mg/tablet |
|---|---|---|
| Active ingredient | | 200 |
| Powd. lactose | | 100 |
| White corn starch | | 64 |
| polyvinylpyrrolidone | | 12 |
| Na carboxymethylstarch | | 20 |
| Magnesium stearate | | 4 |
| | Tablet weight | 400 |

Example C

Capsules of the following composition are produced:

| | | |
|---|---|---|
| Active ingredient | | 50 |
| Cryst. lactose | | 60 |
| Microcrystalline cellulose | | 34 |
| Talc | | 5 |
| Magnesium stearate | | 1 |
| | Capsule fill weight | 150 |

The active ingredient having a suitable particle size, the crystalline lactose and the microcrystalline cellulose are homogeneously mixed with one another, sieved and thereafter talc and magnesium stearate are admixed. The final mixture is filled into hard gelatine capsules of suitable size.

## Claims

**1.** The use of compounds of the general formula

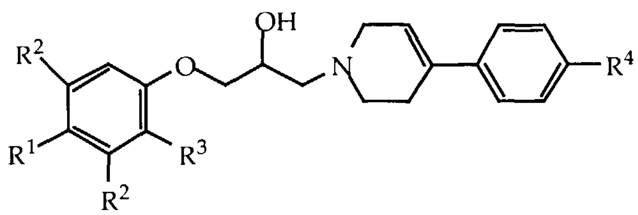

I

wherein

| | |
|---|---|
| $R^1$ | signifies hydrogen, $C_1$-$C_6$-alkyl, halogen, $C_1$-$C_6$-alkoxy or nitro; |
| $R^2$ | signifies hydrogen, $C_1$-$C_6$-alkyl or trifluoromethyl; and |
| $R^3$ | signifies hydrogen, $C_1$-$C_6$-alkyl, trifluoromethyl, benzyl, hydroxy-$C_1$-$C_6$-alkyl, $C_1$-$C_6$-alkoxy, $C_1$-$C_6$-alkyl-carbonyl-amino, carbonyl-$C_1$-$C_6$-alkyl, benzyl, di-$C_1$-$C_6$-alkyl-amino-carbonyl, carbonyl-amino or amino-carbonyl-amino; or |
| $R^2$ and $R^3$ | together optionally signify a fused benzene ring and |
| $R^4$ | signifies hydrogen or halogen, |

and of pharmaceutically acceptable salts of compounds of general formula I for the manufacture of medicaments for treating or preventing psychotic illnesses which are caused by disorders of the dopamine system.

**2.** The use in accordance with claim 1 of compounds of general formula I defined in claim 1 in which $R^1$ signifies hydrogen, $R^2$ signifies hydrogen or trifluoromethyl, $R^3$ signifies hydrogen, carbonyl-amino or acetyl-amino and $R^4$ signifies fluorine or chlorine.

**3.** The use of 1-[4-(4-fluoro-phenyl)-3,6-dihydro-2H-pyridin-1-yl)-3-(3-trifluoromethyl-phenoxy)-propan-2-ol in accordance with claim 1.

**4.** The use of N-[2-[3-[4-(4-chloro-phenyl)-3,6-dihydro-2H-pyridin-1-yl]-2-hydroxypropoxy]-phenyl]-acetamide in accordance with claim 1.

**5.** The use of 2-[3-[4-(4-fluoro-phenyl)-3,6-dihydro-2H-pyridin-1-yl]-2-hydroxypropoxy]-benzamide in accordance with claim 1.

**6.** The use in accordance with any one of claims 1-5 in schizophrenia.

**7.** The use in accordance with any one of claims 1-6 with a reference to a dosage of 1 mg to 1000 mg per day.

## Patentansprüche

**1.** Verwendung von Verbindungen der allgemeinen Formel

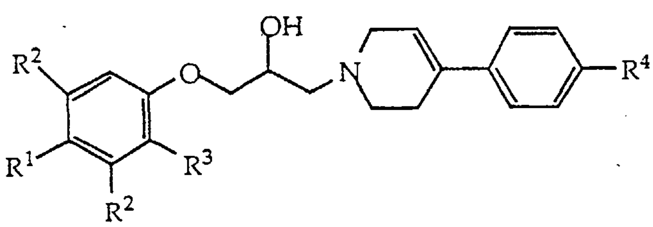

I

in der

| | |
|---|---|
| $R^1$ | ein Wasserstoffatom, einen $C_{1-6}$-Alkyhest, ein Halogenatom, einen $C_{1-6}$-Alkoxyrest oder eine Nitrogruppe darstellt; |
| $R^2$ | ein Wasserstoffatom, einen $C_{1-6}$-Alkylrest oder eine Trifluormethylgruppe darstellt; und |
| $R^3$ | ein Wasserstoffatom, einen $C_{1-6}$-Alkylrest, eine Trifluormethyl-, Benzyl-, Hydroxy-$C_{1-6}$-alkyl-, $C_{1-6}$-Alkoxy-, $C_{1-6}$-Alkylcarbonylamino-, Carbonyl-$C_{1-6}$-alkyl-, Benzyl-, Di-$C_{1-6}$-alkylaminocarbonyl-, Carbonylamino- oder Aminocarbonylarninogruppe darstellt; oder |
| $R^2$ und $R^3$ | zusammen gegebenfalls einen kondensierten Benzolring darstellen und |
| $R^4$ | ein Wasserstoffatom oder ein Halogenatom darstellt, |

und von pharmazeutisch verträglichen Salzen der Verbindungen der allgemeinen Formel I für die Herstellung von Medikamenten zur Behandlung oder Prophylaxe von psychischen Krankheiten, die durch Störungen des Dopaminsystems hervorgerufen werden.

2. Verwendung nach Anspruch 1 von Verbindungen der allgemeinen Formel I wie in Anspruch 1 definiert, in der $R^1$ ein Wasserstoffatom, $R^2$ ein Wasserstoffatom oder eine Trifluormethylgruppe, $R^3$ ein Wasserstoffatom, eine Carbonylamino- oder Acetylaminogruppe und $R^4$ ein Fluor- oder Chloratom darstellt.

3. Verwendung von 1-[4-(4-Fluorphenyl)-3,6-dihydro-2H-pyridin-1-yl]-3-(3-trifluormethylphenoxy)-propan-2-ol nach Anspruch 1.

4. Verwendung von N-[2-[3-[4-(4-Chlorphenyl)-3,6-dihydro-2H-pyridin-1-yl]-2-hydroxypropoxy]-phenyl]-acetamid nach Anspruch 1.

5. Verwendung von 2-[3-[4-(4-Fluorphenyl)-3,6-dihydro-2H-pyridin-1-yl]-2-hydroxypropoxy]-benzamid nach Anspruch 1.

6. Verwendung nach einem der Ansprüche 1 bis 5 bei Schizophrenie.

7. Verwendung nach einem der Ansprüche 1 bis 6, basierend auf einer Dosis von 1 mg bis 1000 mg pro Tag.

## Revendications

1. Utilisation de composés de formule générale

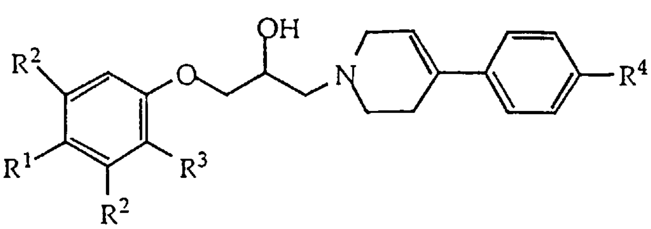

I

dans laquelle

$R^1$ est un atome d'hydrogène, un groupe alkyle en $C_1$-$C_6$, halogéno, alcoxy en $C_1$-$C_6$ ou nitro ;
$R^2$ est un atome d'hydrogène, un groupe alkyle en $C_1$-$C_6$ ou trifluorométhyle ; et
$R^3$ est un atome d'hydrogène, un groupe alkyle en $C_1$-$C_6$, trifluorométhyle, benzyle, hydroxyalkyle en $C_1$-$C_6$, alcoxy en $C_1$-$C_6$, (alkyle en $C_1$-$C_6$)carbonylamino, carbonyl-(alkyle en $C_1$-$C_6$), benzyle, di(alkyle en $C_1$-$C_6$) aminocarbonyle, carbonylamino ou aminocarbonylamino ; ou encore
$R^2$ et $R^3$ forment ensemble éventuellement un noyau benzénique condensé, et
$R^4$ est un atome d'hydrogène ou d'halogène,

et de sels acceptables d'un point de vue pharmaceutique de composés de formule générale I, pour préparer

des médicaments destinés au traitement ou à la prévention des maladies psychotiques qui sont provoquées par des troubles du système dopaminergique.

**2.** Utilisation selon la revendication 1 de composés de formule générale I selon la revendication 1, dans laquelle $R^1$ est un atome d'hydrogène, $R^2$ est un atome d'hydrogène ou le groupe trifluorométhyle, $R^3$ est un atome d'hydrogène ou un groupe carbonylamino ou acétylamino, et $R^4$ est le fluor ou le chlore.

**3.** Utilisation de 1-[4-(4-fluorophényl)-3,6-dihydro-2H-pyridine-1-yl]-3-(3-trifluorométhyl-phénoxy)-propane-2-ol selon la revendication 1.

**4.** Utilisation de N-[2-[3-[4-(4-chloro-phényl)-3,6-dihydro-2H-pyridine-1-yl]-2-hydroxypropoxy]-phényl]-acétamide selon la revendication 1.

**5.** Utilisation de 2-[3-[4-(4-fluorophényl)-3,6-dihydro-2H-pyridine-1-yl]-2-hydroxypropoxy]-benzamide selon la revendication 1.

**6.** Utilisation selon l'une quelconque des revendications 1-5 en présence d'une schizophrénie.

**7.** Utilisation selon l'une quelconque des revendications 1-6, par référence à une posologie de 1 mg à 1000 mg par jour.